# EUROPÄISCHE PATENTSCHRIFT

(11) **EP 3 620 562 B1**
(45) Veröffentlichungstag und Bekanntmachung des Hinweises auf die Patenterteilung: **28.05.2025**
(21) Anmeldenummer: 18020439.8
(22) Anmeldetag: 10.09.2018
(51) Int. Cl.: D04B 1/26, A61F 13/08

(54) **GESTRICK**
KNITTED FABRIC
TEXTILE TRICOTÉ

(43) Veröffentlichungstag der Anmeldung: 11.03.2020
(73) Patentinhaber: medi GmbH & Co. KG, 95448 Bayreuth (DE)
(72) Erfinder: KOLB, Uwe, 94526 Metten (DE)

(56) Entgegenhaltungen:
- WO-A1-98/39982
- WO-A2-2005/094738
- DE-C1- 4 234 379
- US-A- 5 319 807
- US-A- 5 555 565
- US-A1- 2008 286 513

## Beschreibung

Die vorliegende Erfindung bezieht sich auf ein Gestrick, insbesondere auf ein kompressives Gestrick, nach dem Oberbegriff des Anspruch 1.

Derartige Gestricke, vorzugsweise in Form von Kompressionsstrümpfen, finden insbesondere Einsatz im medizinischen Bereich aber auch für die Anwendung bei sportlichen Aktivitäten. Bei der Ausbildung der Gestricke als kompressive Gestricke dienen diese dazu gezielt Druck auf den Körper eines Patienten auszuüben. Der auf den Körper eines Patienten ausgeübte Druck wird als Kompression bezeichnet. Ziel bei Kompressionsstrümpfen beispielsweise ist es unter anderem ein geschädigtes Venen- und/ oder Lymphsystem eines Patienten zu entlasten. Durch den zugeführten Druck wird eine zunehmende Schwellung der Gliedmaßen vermieden, der Abtransport von venösem Blut und Lymphe verbessert und die Blutzufuhr gesteigert. Bei Einsatz von Kompressionsstrümpfen in dem Sportbereich bewirken diese eine Leistungssteigerung bzw. eine verbesserte Regeneration. Insbesondere durch die Wadenmuskulatur (Musculus gastrocnemius), welche bei jeder Aktivität von innen gegen den kompressiven Strumpf mit hoher Stützleistung drückt, werden diese Effekte erzielt.

Zur Ausbildung dieser Gestricke werden diese mittels einer Rundstrickmaschine oder mittels einer ein vorderes und ein hinteres Nadelbett aufweisenden Flachstrickmaschine flach- oder rundgestrickt. Zur Herstellung, insbesondere zur Messung und Gütesicherung, von kompressiven Arm- oder Beinstrümpfen für die medizinische Anwendung, existiert die Norm RAL-GZ 387 der Gütezeichengemeinschaft. Aus den Prüfbestimmungen der RAL kann entnommen werden, wie der Druck eines Kompressionsstrumpfes auf ein Bein zu bestimmen ist. Als Messmittel, insbesondere Kompressionsprüfgerät, wird die Prüfung am HOSY Messgerät (Institut Hohenstein) vorgeschlagen. Die Prüfung erfolgt anhand der Messung der Spannkraft in den mehreren Messpunkten, welche abhängig von der jeweiligen Dehnbarkeit des Gestricks, also abhängig von der Elastizität des Gestricks, variiert. Aus der Spannkraft wird die Kompression errechnet. D.h. je mehr Elastizität ein Gestrick in Gestrickquerrichtung aufweist, desto geringer ist die durch das Gestrick am Patienten ausgeübte Kompression. Andererseits je geringer die Elastizität des Gestrickteils ist, desto höher ist die am Patienten ausgeübte Kompression.

Aus dem Stand der Technik sind eine Vielzahl von Gestricke, insbesondere kompressive Gestricke, für den medizinischen Bereich oder für die Anwendung bei sportlichen Aktivitäten bekannt. Ein Gestrick, insbesondere in Form eines Kompressionsstrumpfes mit temperaturregulierenden Eigenschaften, welches insbesondere den Transport von Feuchtigkeit in das Gestrick berücksichtigt und verbessert, ist beispielsweise aus der DE 20 2010 013 665 U1 bekannt.

Dieser bekannte medizinische Kompressionsstrumpf wird durch einen elastischen Strickfaden gebildet, welcher eine Seele mit mehreren Umwindungen aufweist. Durch die mehreren Umwindungen werden Zwischenräume, insbesondere zwischen den mehreren Filamenten bzw. Fasern, gebildet, welche durch diese eine Sogwirkung erzeugen und somit einen Feuchtetransport bewirken. Gleichzeitig wird durch diesen bekannten Aufbau des elastischen Strickfadens und des dadurch gebildeten Gestricks die Speicherung von Feuchtigkeit in dem Faden und somit in dem Gestrick verbessert. Durch die Ausbildung von mehreren Umwindungen, speichert zumindest die innere Umwindung die Feuchtigkeit, d. h. die Feuchtigkeit wird nicht vollständig abtransportiert. Ziel dieses bekannten Gestricks ist es somit, die Feuchtigkeit in dem Gestrick zu speichern, so dass die Haut eines Trägers nicht austrocknet.

Aus der DE 4234379 C1 ist eine gestrickte Sportsocke bekannt, wobei als Grundstrickfaden ein Garn, welches aus feuchtigkeitstransportierenden Fasern besteht, verwendet wird, um einen Transport von Feuchtigkeit in bzw. durch das Gestrick zu verbessern. Gleichzeitig umfasst das Gestrick mit einem oder mehreren Zusatzgarnen gestrickte Plüschlinge, um einen Ballenschutz auszubilden und die Bildung von Blasen am Fußballen zu vermeiden.

Die US 5,319,807 offenbart eine feuchtigkeitsregulierende Socke, welche aus einem Grundgarn gestrickt ist, auf welches ein erstes Garn mit hydrophoben und ein zweites Garn mit hydrophilen Eigenschaften auf den jeweils gegenüberliegenden Seiten des Grundgarns aufplattiert ist. Hierdurch wird neben einem guten Feuchtigkeitstransport durch das Gestrick auch eine gute Feuchtigkeitsaufnahme gewährleistet.

Die US 5,555,565 sowie WO2005/094738 A2 offenbaren die Verwendung von einem Mischgarn, insbesondere die Kombination von Baumwoll- und/ oder Wollefasern mit feuchtigkeitstransportierenden Fasern, antibakteriellen Silberfasern und Lycra-faser, zur Herstellung von Gestricken für Socken, um den Feuchtigkeitstransport in bzw. durch das Gestrick zu verbessern. Hierbei wird das Mischgarn ebenfalls zumindest bereichsweise derart verstrickt, dass dieses Plüschhenkel bildet, insbesondere um die Feuchtigkeitsaufnahme zu verbessern, aber auch um dem Gestrick polsternde Eigenschaften zu verleihen.

Auch die US 2008/0286513 A1 offenbart ein Gestrick, insbesondere in Form einer Socke, welche zum Transport von Feuchtigkeit in bzw. durch das Gestrick ein Garn, insbesondere ein Mischgarn, bestehend aus feuchtigkeitstransportierenden Fasern, verwendet. Das Mischgarn besteht bevorzugt aus Nylon in Kombination mit Polyester oder Wolle. Auf das Mischgarn sind ein oder mehrere Zusatzfäden aufplattiert, um zusätzlich die Haltbarkeit der Socke zu verbessern.

Als nachteilig der aus dem Stand der Technik bekannten Ausgestaltungen des Gestricks, insbesondere des zur Bildung des Gestricks verwendeten elastischen Strickfadens, erweist sich der nur sehr beschränkt mögliche Feuchtigkeitstransport durch das Gestrick. Insbesondere bei der Ausbildung der Gestricke als Bekleidungsstücke für den Einsatz im Sportbereich ist die Notwendigkeit Feuchtigkeit von der Haut des Trägers, insbesondere von dessen Fußsohle, weg zu transportieren weitaus höher. Hier reichen die bekannten Ausgestaltungen des Strickfadens und somit des Gestricks, insbesondere mit Zwischenräumen zwischen mehreren Fasern eines Umwindegarns eines Strickfadens zur Ausbildung von Kanälen zur Feuchtigkeitsaufnahme, nicht aus, einen ausreichenden Feuchtigkeitstransport zu gewährleisten. Dies hat zur Folge, dass auch bei Verwendung der aus dem Stand der Technik bekannten Maßnahmen, insbesondere für den Sportbereich, nur ein unzureichendes Feuchtigkeitsmanagement bereitgestellt wird.

Einen weiteren Nachteil der aus dem Stand der Technik bekannten Ansätze zur Bereitstellung eines klimaregulierenden Gestricks bildet die gute Speichermöglichkeit der Feuchtigkeit in dem Gestrick. Wie bereits zuvor erwähnt, bestehen, insbesondere bei der Verwendung der Gestricke im Sportbereich, ein weitaus höherer Bedarf an Feuchtigkeitstransport, da durch die körperlichen Anstrengungen des Trägers wesentlich mehr Feuchtigkeit produziert wird, welche von der Haut des Trägers weg in und vorzugsweise durch das Gestrick nach außen weg transportiert werden muss. Die Speicherfähigkeit der Feuchtigkeit stellt somit einen wesentlichen Nachteil dar und ist, insbesondere im Sportbereich, nicht gewünscht.

Folglich stellt somit auch die fehlende Berücksichtigung bzw. Verbesserung der Abgabe der in dem Gestrick aufgenommenen Feuchtigkeit, einen Nachteil dar. Neben der Aufnahme der Feuchtigkeit in dem Gestrick, dem Transport der Feuchtigkeit durch das Gestrick, stellt insbesondere die Abgabe dieser einen wesentlichen Bestandteil des Feuchtigkeitsmanagements eines Gestricks dar.

Der vorliegenden Erfindung liegt daher die Aufgabe zugrunde ein Gestrick, insbesondere ein klimaregulierendes und vorzugsweise kompressives Gestrick, zu schaffen, welches die Nachteile aus dem Stand der Technik vermeidet, insbesondere welches die Aufnahme von Feuchtigkeit, den Feuchtigkeitstransport durch das Gestrick, sowie die Abgabe der Feuchtigkeit nach außen verbessert.

Erfindungsgemäß besteht das Gestrick aus zumindest einem maschenbildenden Grundstrickfaden, mit zumindest einem auf den Grundstrickfaden plattierten Plüschhenkel ausbildenden Plüschfaden, wobei der zumindest eine Grundstrickfaden aus mehreren miteinander versponnen Profilfasern, welche eine strukturierte Oberfläche mit Erhebungen und Vertriefungen aufweisen, besteht, um zwischen den mehreren Fasern Kanäle zum Transport von Feuchtigkeit auszubilden und wobei sich der Plüschfaden durch das Gestrick erstreckt und an beiden Seiten des Gestricks hervortritt.

Gemäß einem weiteren Ausführungsbeispiel des Gestricks besteht der zumindest eine maschenbildende Grundstrickfaden aus mehreren miteinander versponnen Profilfasem und der zumindest eine Plüschfaden, sowie ein optionaler Verstärkungsfaden, aus mehreren Fasern mit im Wesentlich rundlichen Querschnitten, wobei die Fäden derart miteinander verstrickt sind, dass der Plüschfaden im Wesentlichen beide Seiten des Gestricks bildet.

Dabei erstreckt sich der Plüschfaden durch das Gestrick und tritt an beiden Seiten des Gestricks hervor, insbesondere mit dessen Plüschhenkel an der einen Seite und mit dessen Maschenfüßen an der anderen Seite. Hierdurch wird die Feuchtigkeitsaufnahme des Gestricks weiter optimiert.

Durch diesen mehrschichtigen Aufbau des Gestrickes

Des Weiteren ist der zumindest eine Plüschfaden bevorzugt jeweils nur abschnittsweise, in Umfangsrichtung verlaufend, in das Gestrick eingebracht, so dass die feuchtigkeitsregulierende Wirkung des Gestricks nur lokal eingesetzt wird, insbesondere an Stellen in denen sie benötigt wird. In anderen Bereichen kann das Gestrick beispielsweise nur mit dem Grundstrickfaden ausgebildet sein, so dass dort ein wesentlich dünneres Gestrick vorliegt.

Gemäß einem weiteren Ausführungsbeispiel ist zumindest ein elastischer Schussfaden in das Gestrick eingelegt und/ oder verstrickt. Bevorzugt ist der zumindest eine Schussfaden hierbei derart mit den mehreren Fäden des Gestricks verstrickt, dass dieser in Gestrickumfangsrichtung in jedem n-ten Maschenstäbchen an einer der beiden Seiten des Gestricks hervortritt.

Bevorzugt bestehen die Profilfasern des maschenbildenden Grundstrickfadens, aus Kunststoff, insbesondere Polyamid, Polyester, Polypropylen oder Polyurethan. Alternativ ist es möglich, dass die Fasern mit im Wesentlichen runden Querschnitten des Plüschfadens aus Naturfasern, insbesondere Wolle oder Zellulose bzw. Viskose, bestehen.

Gemäß einem weiteren Ausführungsbeispiel sind die Profilfasern des maschenbildenden Grundstrickfadens, mit einer Garndrehung, vorzugsweise größer als 80 Touren pro Meter (T/m) besonderes bevorzugt größer als 150 Touren pro Meter (T/m), versehen. Durch diese deutlich erhöhte Beaufschlagung der Fasern mit mehr Garndrehungen kommt es, bei den Profilfasern zu einer wesentlich besseren Ausbildung von Kanälen, da die Fasern näher zusammenrücken. Dies hat zur Folge, dass die Sogwirkung der Fasern und somit des Gestricks deutlich verbessert wird.

Gemäß einem weiteren Ausführungsbeispiel weist der zumindest eine maschenbildenden Grundstrickfaden, der elastische Schussfaden, der Verstärkungsfaden und/ oder Plüschfaden einen elastischen Fadenkern, vorzugsweise aus Elastan, auf.

Das Gestrick ist bevorzugt als Strumpf, insbesondere als Arm- oder Beinstumpf, Socke, Beinling, Armling, Legging oder Hose, Bandage, oder als ein Strickteil einer Orthese ausgebildet. Besonders bevorzugt ist es als ein kompressives Gestrickteil ausgebildet, wobei die Kompressionsstärke in Gestricklängsrichtung bevorzugt ab oder zunimmt.

Bei Ausbildung des Gestricks als Beinbekleidungsstück, insbesondere als Beinstrumpf oder Socke, ist der zumindest eine die Plüschhenkel bildenden Plüschfaden derart in dem Gestrick eingestrickt, dass dieser im getragenen Zustand des Gestricks im Bereich der Achillessehne, Ferse, Zehen und/ oder der Fußsohle an der Gestrickinnenseite angeordnet ist. Im Falle eines kompressiven Beinbekleidungsstücks, betragen die durch das Gestrickteil erzeugten kompressiven Drücke in einem Fesselbereich des Beinbekleidungsstücks bevorzugt zwischen 10 und 40 mmHg, im Wadenbereich bevorzugt zwischen 5 und 30 mmHg und in einem Mittelfußbereich bevorzugt zwischen 10 und 30 mmHg.

Das vorliegende Beinbekleidungsstück zeichnet sich durch eine Reihe erheblicher Vorteile aus.

Durch die Ausbildung des, vorzugsweise kompressiven, Gestricks mit oder ohne Schussfaden, sowie mit mehreren Strickfäden, wobei zumindest einer von diesen aus mehreren miteinander versponnen Profilfasern besteht, um zwischen den mehreren Fasern Kanäle zum Transport von Feuchtigkeit auszubilden, wird ein deutlich verbesserter Feuchtetransport gewährleistet. Insbesondere die Erhebungen und Vertiefungen in den Oberflächen der mehreren Fasern bzw. Filamente bevorzugt in Kombination mit der erhöhten Garndrehung verleihen dem Faden optimale Feuchtetransporteigenschaften.

Einen weiteren Vorteil bildet die Kombination eines Fadens, bestehend aus Profilfasern, mit einem oder mehreren Fäden, welche aus Fasern mit im Wesentlich rundlichen Querschnitten bestehen. Letzt genannter Faden weist eine sehr große Kontaktfläche zur Anlage an der Haut eines Trägers auf, so dass neben einer guten Leitfähigkeit durch den Faden mit Profilfasern, auch eine besonders gute Feuchtigkeitsaufnahme durch das Gestrick gewährleistet wird. Das Zusammenspiel zwischen Feuchtigkeitsaufnahme und Transport durch das Gestrick ist besonders wichtig für die klimaregulierende Ausbildung des, vorzugsweise für den Sportbereich ausgelegten, Gestricks.

Es wird ferner durch einen weiteren Faden mit Fasern, welche einen rundlichen Querschnitt aufweist, und welcher bevorzugt, gesehen vom Träger aus, an der Gestrickaußenseite angeordnet ist und hierbei eine weitere Schicht in dem mehrschichtigen Aufbau des Gestrickes bildet, eine besonders gute Feuchtigkeitsregulierung, ermöglicht. Der außenliegende Faden weist gute Feuchtigkeitsaufnahme- und Abgabeeigenschaften auf. Er nimmt die durch das Gestrick und durch die Profilfasern geleitete Feuchtigkeit gut auf und kann diese gut an das Umfeld abgeben.

Einen weiteren Vorteil der Erfindung bildet die Einarbeitung eines elastischen Schussfadens in das Gestrick, welcher neben der Verleihung von erhöhten kompressiven Eigenschaften derart in das Gestrick eingearbeitet ist, dass er besondere bevorzugt zumindest teilweise alle drei Fäden an die Haut eines Trägers drückt. Hierdurch wird ein besonders guter Kontakt zwischen den mehreren Fäden und zwischen dem Gestrick und der Haut eines Trägers sichergestellt, was dem Gestrick eine besonders hohe Feuchtigkeitsregulierung verleiht.

Einen weiteren Vorteil bildet neben der Feuchteregulierung auch die gute temperaturregulierende Wirkung des Gestricks. Durch den mehrschichtigen Aufbau und der mehreren Kanäle in den Fäden entsteht ein atmungsaktives Gestrick.

Nachfolgend wird die Erfindung anhand mehrerer Ausführungsbeispiele und in Verbindung mit den beigefügten Zeichnungen erläutert.

Dabei zeigt:
Figur 1 ein Ausführungsbeispiel des erfindungsgemäßen Gestricks, wobei das Gestrick als Strumpf, insbesondere als Beinstumpf, ausgebildet ist,
Figur 2 ein Maschenbild, insbesondere einen ersten Ausschnitt aus dem in Figur 1 gezeigten Ausführungsbeispiel des Gestricks,
Figur 3 ein Maschenbild, insbesondere einen zweiten Ausschnitt aus dem in Figur 1 gezeigten Ausführungsbeispiel des Gestricks,
Figur 4A einen Grundstrickfaden, Verstärkungsfaden und/ oder Plüschfaden aus mehreren miteinander versponnen Fasern, in einer vergrößerten Detailansicht, sowie
Figur 4B den Faden aus Figur 4A in einer Schnittdarstellung mit den mehreren durch die Profilfasern gebildeten Kanälen zum Transport von Feuchtigkeit.

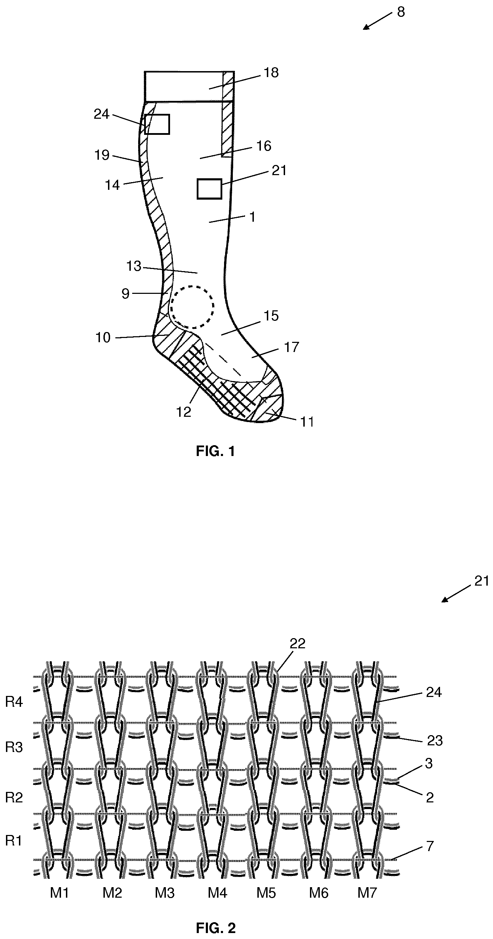

In Figur 1 ist ein erstes Ausführungsbeispiel des erfindungsgemäßen Gestricks 1, wobei das Gestrick 1 als Strumpf 8, insbesondere als Beinstumpf, ausgebildet ist, gezeigt. Der Strumpf 8, insbesondere Sportstrumpf, besteht aus zumindest einem ersten und zweiten bevorzugt mittels eine Rundstrickmaschine gestrickten Gestrickabschnitt 16, 17. Am oberen Ende des ersten Abschnitts 16 weist der Strumpf 8 einen zweilagigen Bund 18 auf. Die durch das Gestrick 1 erzeugten kompressiven Drücke betragen in einem Fesselbereich 13 des Beinbekleidungsstücks bevorzugt zwischen 10 und 40 mmHg, im Wadenbereich 14 zwischen 5 und 30 mmHg und in einem Mittelfußbereich 15 zwischen 10 und 30 mmHg. Die Werte werden mittels der eingangs vorgestellten Messmethodik und Messgerät, insbesondere mittels der Prüfung am HOSY Messgerät (Institut Hohenstein), gemessen.

An der Innenseite weist der Strumpf 8 eine erste Polsterung 19 auf. Die Polsterung 19 erstreckt sich hierbei bevorzugt in Umfangsrichtung gesehen nur zwischen den beiden Muskelköpfen der Wadenmuskulatur ohne diese zu überlappen. Die Polsterung 19 dient dazu eine von einem kreisförmigen Querschnitt abweichende Geometrie eines Beines auszugleichen. Ferner erstreckt sich die zumindest eine Polsterung 19, wie gezeigt, im getragenen Zustand des Strumpfes 8 zusätzlich zumindest teilweise entlang der Achillessehne 9 in Richtung Ferse 10 und überlappt die Sehne.

Des Weiteren weist der Strumpf 8 ein Fußteil auf, welches aus einem zweiten, vorzugsweise kompressiven, Gestrickteil 17, besteht. Dieses Fußteil 17 umfasst eine weitere Polsterung 20 für den Zehenbereich 11, die Ferse 10 und die Fußsohle 12. Die Polsterung 20 erstreckt sich in diesem Ausführungsbeispiel von der Sohle 12 bis in die Seiten des Fußteils 17 hinauf. Die Polsterung 20 kann auch nur in einzelnen Abschnitten der zuvor genannten Bereiche angeordnet sein.

In diesem Ausführungsbeispiel sind die mehreren Polsterungen 19 und 20 durch einen gestrickten Abschnitt in den Gestrick 1 ausgebildet, der sich bezüglich seiner Bindungsart von einem an die Polsterung 19, 20 angrenzenden Bereich der Grundgestricke 1 unterscheidet. Hierbei sind die mehreren Polsterungen 19, 20 erfindungsgemäß als Sandwich-Plüsch ausgebildet. Das Gestrick 1 sowie die erste Polsterung 19 weist in dem gezeigten Ausführungsbeispiel in Gestricklängsrichtung einen graduellen Kompressionsverlauf auf. Die Kompressionsstärke nimmt hierbei vom unteren Ende in Richtung der Wadenmuskulatur ab. Der graduelle Druckverlauf wird durch die Art und Weise der Einbringung des Schussfadens 7 bestimmt, insbesondere über die Anzahl von aufeinanderfolgenden Maschenreihen in den der Schussfaden 7 eingelegt ist. Alternativ ist es natürlich möglich, dass das erfindungsgemäße Gestrick 1 als Armstumpf, Socke, Bandage, oder ein Strickteil einer Orthese ausgebildet ist.

Ein Maschenbild, insbesondere ein erster Ausschnitt 21 aus dem in Figur 1 gezeigten Ausführungsbeispiel des Gestricks 1, ist in Figur 2 gezeigt. Das Maschenbild zeigt einen Grundstrickfaden 2, der maschenbildend über mehrere Strickreihen R1-R4 und Maschenstäbchen M1-M7 hinweg verstrickt ist, in den zumindest ein kompressionsgebender Schussfaden 7 eingelegt ist. Der hochelastische Schussfaden 7 ist in diesem Ausführungsbeispiel in jeder Maschenreihe R1-R4 eingelegt. Dies kann auch nur wahlweise in jeder beliebigen Reihe erfolgen. Des Weiteren kann dieser Faden 7 zumindest abschnittsweise in Umfangsrichtung gesehen in einer oder mehreren Reihen M1-M7 maschenbildend verstrickt sein. Neben dem Grundstrickfaden 2 und dem Schussfaden 7 zeigt das Maschenbild einen auf den Grundstrickfaden 2 plattierten Verstärkungsfaden 3. Dieser ist gemäß diesem Ausführungsbeispiel in jeder Maschenreihe R1-R4 und jedem Maschenstäbchen M1-M7 vorhanden. Auch dieser Faden 3 kann nur in jeder beliebigen Maschenreihe und nur abschnittsweise in Umfangsrichtung gesehen, in das Gestrick 1 eingestrickt sein. In der Zeichnung ist der Verstärkungsfaden 3 neben dem Grundstrickfaden 2 abgebildet, dies aber nur zu Zwecke der Veranschaulichung. Gemäß der stricktechnischen Einbindung, nämlich das Plattieren des Verstärkungsfadens 3 auf den Grundstrickfaden 2, befindet sich der Verstärkungsfaden 3 im Wesentlichen vor oder hinter dem Grundstrickfaden 2, bei einer Draufsicht auf das Gestrick 1. Die Fäden 2, 3 liegen also im Gestrick 1 im Wesentlichen, also größtenteils, über- bzw. hintereinander, so dass die zumindest zwei Fäden 2, 3 derart miteinander verstrickt sind, dass der Grundstrickfaden 2 im Wesentlichen die eine Seite, bevorzugt die der Haut des Trägers zugewandten Seite, und der Verstärkungsfaden 3 im Wesentlichen die andere Seite des Gestricks 1 bildet. Gemäß der Maschenbildung sind bevorzugt jeweils die Maschenköpfe 22 und Maschenfüße 23 des Grundstrickfadens 2 und des Verstärkungsfadens 3 der Haut des Trägers zugewandt. Die Maschenschenkel 24 befinden sich im Gestrickinneren.

Durch das Einstricken bzw. Einlegen des Schussfadens 7, insbesondere über Fang und Flottung, werden der Grundstrickfaden 2 sowie der Verstärkungsfaden 3 besonders stark an die Haut eines Trägers gedrückt, so dass ein besonders guter Kontakt zwischen den mehreren Fäden 2, 3 und zwischen dem Gestrick 1 und dem Träger zustande kommt, was zusätzlich die Funktion des Gestricks 1 verbessert. Der Schussfaden 7 tritt in Gestrickumfangsrichtung gesehen in jedem n-ten Maschenstäbchen an einer der beiden Seiten des Gestricks 1 hervor.

Die Fasern der mehreren Fäden 2, 3 bestehen in dem bevorzugten Ausführungsbeispiel bevorzugt aus Kunststoff, insbesondere Polyamid, Polyester oder Polyurethan.

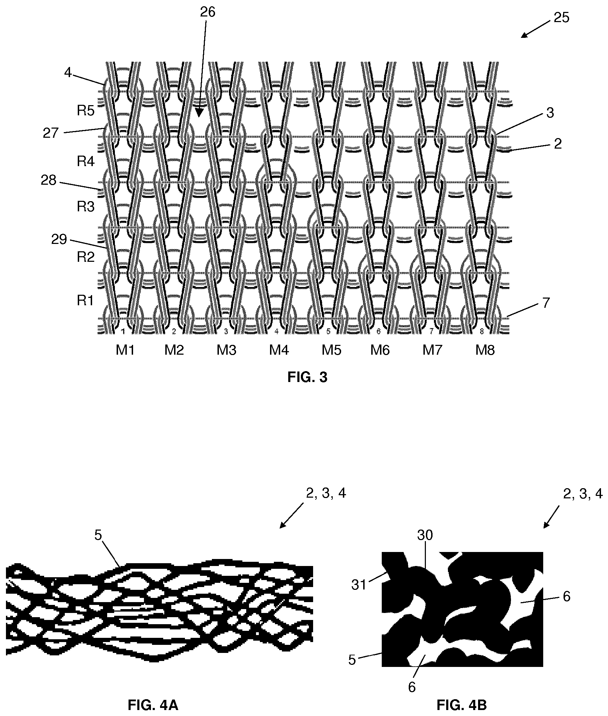

In Figur 3 ist nun ein zweiter Ausschnitt 25, in Form eines Maschenbildes, aus dem in Figur 1 gezeigten Strumpf 8, insbesondere Sportstrumpf, gezeigt. Neben dem Verstärkungsfaden 3 ist in einem Abschnitt 26 des Gestricks 1 des Weiteren, also zusätzlich im Vergleich zu Abschnitt 21, ein Plüschfaden 4 auf den Grundstrickfaden 2 plattiert. D.h. der Plüschfaden 4 ist zusätzlich zu dem Grundstrickfaden 2, dem Verstärkungsfaden 3 und dem Schussfaden 4 in das Gestrick 1 eingearbeitet. Auch dieser Faden 4 ist zu Veranschaulichungszwecken neben den mehreren Fäden abgebildet. In Wirklichkeit sind die zumindest drei Fäden 2, 3, 4 derart miteinander verstrickt, dass der Plüschfaden 4 im Wesentlichen die eine Seite des Gestricks 1, also die Innenseite des Strumpfes 8, sowie die Außenseite bildet. Der Plüsch ist hierbei nämlich als so genannter Sandwich-Plüsch ausgebildet. So erstrecken sich die Plüschhenkel 27, die an der Innenseite des Gestricks 1 angeordnet sind, von dieser durch das Gestrick 1 bis an dessen Außenseite. Dort treten, wie beschrieben, die Maschenfüße 28 der Plüschmaschen 29 hervor. Der Grundstrickfaden 2 sowie der Verstärkungsfaden 3 befinden sich somit zwischen den durch den Plüschfaden gebildeten Gestrickebenen.

Die verwendeten, insbesondere zumindest teilweise hochelastischen Fäden 2, 3, 4, 7 können sich neben den unterschiedlichen Fasern, insbesondere hinsichtlich Material und Geometrie der Fasern, auch darin unterscheiden, dass diese wahlweise einen Fadenkern aufweisen. Auch die Anzahl der Fasern kann unterschiedlich sein. Ferner können die Fasern eine oder mehrere Umwindungen für einen Fadenkern bilden. Auch können es sich wahlweise um texturierte Fäden handeln.

Figur 4A zeigt nun einen als Grundstrickfaden 2, verwendbaren Faden aus mehreren miteinander versponnen Fasern, in einer vergrößerten Detailansicht. Die Fasern sind Is Profilfasern 5 ausgebildet**.**

Profilfasern 5 können beispielsweise dreieckige, dreilappige, oder sternförmige Querschnitte, besonders bevorzugt in Form eines Trilobals, aufweisen. Weitere Formen sind denkbar. Weist der Faden mehrere Profilfasern 5 auf, enthält dieser vorzugsweise über 50 einzelnen Fasern, welche mit einer Garndrehung, vorzugsweise größer als 80 Touren pro Meter (T/m) besonderes bevorzugt größer als 150 Touren pro Meter, beaufschlagt sind. Hierdurch wird neben der Haltbarkeit des Fadens 3, insbesondere die Position der Fasern 5 zueinander beeinflusst. Die sogenannte Schutzdrehung mit der deutlich erhöhten Anzahl an Garndrehungen (Touren pro Meter) als üblicherweise bekannt, führt zu einem besonders guten Zusammenhalt der Fasern 5, aber auch dazu, dass die Fasern 5 deutlich enger zusammenrücken und folglich, durch die mehreren Erhebungen 30 und/ oder Vertiefungen 31 in den Profilfasern 5, zu einer wesentlich besseren Ausbildung von Kanälen 6. Dies hat zur Folge, dass die Sogwirkung der Fasern 5 und somit des Gestricks 1, durch die erhöhte Garndrehung, deutlich verbessert wird.

Die Fasern 5 können, wie zuvor beschrieben, aus Kunststoff, insbesondere Polyamid, Polyester oder Polyurethan, bestehen.

Zur besseren Veranschaulichung der durch die mehreren Profilfasern 5 gebildeten Kanäle 6 zum Transport von Feuchtigkeit zeigt nun Figur 4B den Faden 2 aus Figur 4A, wenn dieser als Faden bestehend aus mehreren Profilfasern 5 gebildet ist, in einer Schnittdarstellung mit den mehreren durch die Profilfasern 5 gebildeten Kanälen 6 zum Transport von Feuchtigkeit. Zu sehen ist, dass durch die willkürliche Anordnung der mehreren Fasern 5 zueinander sowie durch die Garndrehung die Kanäle 6 zum Transport von Feuchtigkeit entstehen. Diese werden insbesondere dadurch gebildet, dass die Oberfläche der Fasern 5 eine Struktur, nämlich Erhebungen 30 und/ oder Vertiefungen 31, beispielsweise wie gezeigt in Form eines Trilobals, aufweisen. Die entstandenen Hohlräume 6 weisen unterschiedliche Durchmesser und Querschnitte auf, je nachdem wie die benachbarten Fasern 5 aneinander liegen. Je höher die Garndrehung ist, umso besser ist der Kontakt zwischen den Fasern 5, d.h. desto mehr geschlossene Kanäle 6 entstehen. Bei einer niedrigen Garndrehung besteht die Gefahr, dass, in Umfangsrichtung gesehen, viele offene Kanäle 6 entstehen, da die Fasern 5 nicht eng genug aneinander anliegen. Die Kanäle 6 weisen schlussendlich durch die geometrische Form und der erhöhten Garndrehung eine besonders gute Sogwirkung bzw. Kapillarwirkung auf. Als nachteilig erweist sich jedoch, dass Fäden mit derartigen Profilfasern 5, auf Grund der Erhebungen 30 und/ oder Vertiefungen 31 wenig Kontaktfläche zu einer Haut eines Patienten aufweisen. Deswegen ist es besonders vorteilhaft derartige Fäden mit Fäden zu kombinieren, welche einen rundlichen Querschnitt aufweisen.

## Patentansprüche

1. Gestrick (1) bestehend aus zumindest einem maschenbildenden Grundstrickfaden (2) sowie zumindest einem auf den Grundstrickfaden (2) plattierten Plüschhenkel ausbildenden Plüschfaden (4), wobei der zumindest eine Grundstrickfaden (2) aus mehreren miteinander versponnen Profilfasern (5), welche eine strukturierte Oberfläche mit Erhebungen (30) und/ oder Vertiefungen (31) aufweisen, besteht, um zwischen den mehreren Profilfasern (5) Kanäle (6) zum Transport von Feuchtigkeit auszubilden und der zumindest eine Plüschfaden (4) aus mehreren Fasern mit im Wesentlich rundlichen Querschnitten besteht, **dadurch gekennzeichnet, dass** mit dem Plüschfaden (4) ein Sandwich-Plüsch ausgebildet wird, wobei sich die durch den zumindest einer Plüschfaden (4) gebildeten Plüschhenkel durch das Gestrick (1) erstrecken, so dass der Plüschfaden (4) an beiden Seiten des Gestricks (1) hervortritt und der Plüschfaden (4) im Wesentlichen die Innenseite sowie die Außenseite des Gestricks (1) bildet.

2. Gestrick (1) nach Anspruch 1, **dadurch gekennzeichnet, dass** der Plüschfaden (4) jeweils nur abschnittsweise, in Umfangsrichtung verlaufend, in das Gestrick (1) eingebracht ist.

3. Gestrick (1) nach einem der vorherigen Ansprüche, **dadurch gekennzeichnet, dass** zumindest ein elastischer Schussfaden (7) in das Gestrick (1) eingelegt und/ oder verstrickt ist, der vorzugsweise derart mit den mehreren Fäden (2, 4) verstrickt ist, dass dieser in Gestrickumfangsrichtung in jedem n-ten Maschenstäbchen an einer der beiden Seiten des Gestricks (1) hervortritt.

4. Gestrick (1) nach einem der vorherigen Ansprüche, **dadurch gekennzeichnet, dass** die Profilfasern (5) eine strukturierte Oberfläche in Form eines Trilobals aufweisen.

5. Gestrick (1) nach einem der vorherigen Ansprüche, **dadurch gekennzeichnet, dass** die Profilfasern (5) des maschenbildenden Grundstrickfadens (2) aus Kunststoff, insbesondere Polyamid, Polyester oder Polyurethan, bestehen.

6. Gestrick (1) nach Anspruch 1 oder 2, **dadurch gekennzeichnet, dass** die Fasern mit im Wesentlichen rundlichen Querschnitten des Plüschfadens (4) aus Naturfasern, insbesondere Wolle, bestehen.

7. Gestrick (1) nach einem der vorherigen Ansprüche, **dadurch gekennzeichnet, dass** die mehreren Profilfasern (5) mit einer Garndrehung, vorzugsweise größer als 80 Touren pro Meter (T/m) besonderes bevorzugt größer als 150 Touren pro Meter (T/m), versehen sind.

8. Gestrick (1) nach Anspruch 3, **dadurch gekennzeichnet, dass** der zumindest eine maschenbildenden Grundstrickfaden (2), der elastische Schussfaden (7) und/ oder Plüschfaden (4) einen elastischen Fadenkern, vorzugsweise aus Elastan, aufweisen.

9. Gestrick (1) nach einem der vorangehenden Ansprüche, **dadurch gekennzeichnet, dass** das Gestrick (1) ein Strumpf (8), insbesondere ein Arm- oder Beinstrumpf, eine Socke, eine Bandage, oder ein Strickteil einer Orthese ist.

10. Gestrick (1) nach Anspruch 9, **dadurch gekennzeichnet, dass** der zumindest eine die Plüschhenkel bildenden Plüschfaden (4) derart in dem als Beinbekleidungsstück (8) ausgebildete Gestrick (1) eingestrickt ist, dass die Plüschhenkel im getragenen Zustand des Gestricks (1) im Bereich der Achillessehne (9), Ferse (10), Zehen (11) und/ oder der Fußsohle (12) an der Gestrickinnenseite angeordnet sind.

11. Gestrick (1) nach Anspruch 10, **dadurch gekennzeichnet, dass** die durch das Gestrick (1) erzeugten kompressiven Drücke in einem Fesselbereich (13) des Beinbekleidungsstücks (1) zwischen 10 und 40 mmHg, im Wadenbereich (14) zwischen 5 und 30 mmHg und in einem Mittelfußbereich (15) zwischen 10 und 30 mmHg betragen.

## Claims

1. Knitted fabric (1) consisting of at least one stitch-forming base knitting thread (2) and at least one plush thread (4) forming plush loops plated onto the base knitting thread (2), wherein the at least one base knitting thread (2) consists of a plurality of profile fibres (5) spun together, which have a structured surface with elevations (30) and/or depressions (31) in order to form channels for transporting moisture between the plurality of profile fibres (5) and the at least one plush thread (4) consists of a plurality of fibres with substantially round cross-sections, **characterized in that** a sandwich plush is formed with the plush threads (4), wherein the plush loops formed by the at least one plush thread (4) extend through the knitted fabric (1) so that the plush thread (4) protrudes on both sides of the knitted fabric (1) and the plush thread (4) substantially forms the inner side and the outer side of the knitted fabric (1).

2. Knitted fabric (1) according to Claim 1, **characterized in that** the plush thread (4) is introduced into the knitted fabric (1) in each case only in sections, running in the circumferential direction.

3. Knitted fabric (1) according to one of the preceding claims, **characterized in that** at least one elastic weft thread (7) is laid and/or knitted into the knitted fabric (1), which is preferably knitted with the plurality of threads (2, 4) in such a manner that this protrudes in the knitted-fabric (1) circumferential direction in every n-th stitch wale on one of the two sides of the knitted fabric (1).

4. Knitted fabric (1) according to one of the preceding claims, **characterized in that** the profile fibres (5) have a structured surface in the form of a trilobal.

5. Knitted fabric (1) according to one of the preceding claims, **characterized in that** the profile fibres (5) of the stitch-forming base knitted thread (2) consist of plastic, in particular polyamide, polyester or polyurethane.

6. Knitted fabric (1) according to Claims 1 or 2, **characterized in that** the fibres having substantially round cross-sections of the plush thread (4) consist of natural fibres, in particular wool.

7. Knitted fabric (1) according to one of the preceding claims, **characterized in that** the plurality of profile fibres (5) are provided with a yarn twist, preferably greater than 80 turns per metre (T/m), particularly preferably greater than 150 turns per metre (T/m).

8. Knitted fabric (1) according to Claim 3, **characterized in that** the at least one stitch-forming base knitting thread (2), the elastic weft thread (7) and/or plush thread (4) have an elastic thread core, preferably of elastane.

9. Knitted fabric (1) according to one of the preceding claims, **characterized in that** the knitted fabric (1) is a stocking (8), in particular an arm sleeve or leg stocking, a sock, a bandage or a knitted part of an orthesis.

10. Knitted fabric (1) according to Claim 9, **characterized in that** the at least one plush thread (4) forming the plush loop is knitted in the knitted fabric (1) formed as a legwear item (8) in such a manner that in the worn state of the knitted fabric (1), the plush loops are arranged in the area of the Achilles tendon (9), heel (10), toe (11) and/or the sole of the foot (12) on the inner side of the knitted fabric.

11. Knitted fabric (1) according to Claim 10, **characterized in that** the compressive pressures generated by the knitted fabric (1) in an ankle region (13) of the legwear item (1) are between 10 and 40 mmHg, in the calf region (14) between 5 and 30 mmHg and in a metatarsal region (15) between 10 and 30 mmHg.

## Revendications

1. Tricot (1), constitué d'au moins un fil à tricoter (2) de base formant les mailles, ainsi que d'au moins un fil (4) de peluche constituant des bouclettes, plaqué sur le fil à tricoter (2) de base, l'au moins un fil à tricoter (2) de base étant constitué de plusieurs fibres profilées (5) filées les unes avec les autres, lesquelles comportent une surface structurée dotée d'élévations (30) et / ou de creux (31), pour constituer entre les plusieurs fibres profilées (5) des canaux pour le transport d'humidité et l'au moins un fil (4) de peluche étant constitué de plusieurs fibres avec des sections transversales sensiblement arrondies, **caractérisé en ce qu'**avec le fil (4) de peluche est constituée une peluche en sandwich, les bouclettes formées par l'au moins un fil de peluche (4) s'étendant à travers le tricot (1), de telle sorte que le fil (4) de peluche ressorte sur les deux faces du tricot (1) et que le fil (4) de peluche forme essentiellement la face intérieure ainsi que la face extérieure du tricot (1).

2. Tricot (1) selon la revendication 1, **caractérisé en ce que** le fil de peluche (4) n'est introduit chaque fois dans le tricot (1) que par endroits, en s'écoulant dans la direction circonférentielle.

3. Tricot (1) selon l'une quelconque des revendications précédentes, **caractérisé en ce qu'**au moins un fil de trame (7) élastique qui est de préférence tricoté avec les plusieurs fils (2, 4) est inséré et / ou tricoté dans le tricot (1), **en ce que** dans la direction circonférentielle du tricot, celui-ci ressort à chaque n^{ème} colonne de mailles sur l'une des deux faces du tricot (1).

4. Tricot (1) selon l'une quelconque des revendications précédentes, **caractérisé en ce que** les fibres profilées (5) comportent une surface structurée, sous forme trilobale.

5. Tricot (1) selon l'une quelconque des revendications précédentes, **caractérisé en ce que** les fibres profilées (5) du fil à tricoter (2) de base formant les mailles sont constituées de matière plastique, notamment de polyamide, de polyester ou de polyuréthane.

6. Tricot (1) selon la revendication 1 ou 2, **caractérisé en ce que** les fibres de section transversale sensiblement arrondie du fil de peluche (4) sont constituées de fibres naturelles, notamment de laine.

7. Tricot (1) selon l'une quelconque des revendications précédentes, **caractérisé en ce que** les plusieurs fibres profilées (5) sont pourvues d'une torsion de fil, de préférence supérieure à 80 tours par mètre (T/m) de manière particulièrement préférentielle, supérieure à 150 tours par mètre (T/m).

8. Tricot (1) selon la revendication 3, **caractérisé en ce que** l'au moins un fil à tricoter (2) de base formant les mailles, le fil de trame (7) élastique et / ou le fil (4) de peluche comportent un noyau de fil élastique, de préférence en élasthane.

9. Tricot (1) selon l'une quelconque des revendications précédentes, **caractérisé en ce que** le tricot (1) est une chaussette (8), notamment un manchon ou une chaussette, une socquette, un bandage, ou une partie tricotée d'une orthèse.

10. Tricot (1) selon la revendication 9, **caractérisé en ce que** l'au moins un fil (4) de peluche formant les bouclettes est tricoté dans le tricot (1) conçu sous la forme d'une pièce de vêtement (8) pour jambe de telle sorte que lorsque le tricot (1) est porté, les bouclettes soient placées sur la face intérieure du tricot, dans la zone du tendon d'Achille (9), du talon (10), des orteils (11) et / ou de la plante du pied (12) .

11. Tricot (1) selon la revendication 10, **caractérisé en ce que** dans une zone de la cheville (13) de la pièce de vêtement (1) pour jambe, les pressions compressives générées par le tricot (1) se situent entre 10 et 40 mmHg, dans la zone du mollet (14), entre 5 30 mmHg et dans la zone du métatarse (15) entre 10 et 30 mmHg.
